# EUROPÄISCHE PATENTSCHRIFT

(11) **EP 3 546 589 B1**
(45) Veröffentlichungstag und Bekanntmachung des Hinweises auf die Patenterteilung: **10.08.2022**
(21) Anmeldenummer: 19163357.7
(22) Anmeldetag: 18.03.2019
(51) Int. Cl.: C12P 13/02, C07H 15/00, A61Q 19/00, A61Q 5/00, A61Q 5/02, A61Q 5/12, A61K 8/68

(54) **VERFAHREN ZUR HERSTELLUNG VON SPHINGOLIPIDEN**
METHOD FOR THE PREPARATION OF SPHINGOLIPIDS
PROCÉDÉ DE PRODUCTION DE SPHINGOLIPIDES

(30) Priorität: 29.03.2018 EP 18164835
(43) Veröffentlichungstag der Anmeldung: 02.10.2019
(73) Patentinhaber: Evonik Operations GmbH, 45128 Essen (DE)
(72) Erfinder: Eckstein, Marrit Friederike, 45289 Essen (DE); van Logchem, Monica Desiree, 4762 PA Zevenbergen (NL); Wenk, Hans Henning, 45470 Mülheim an der Ruhr (DE); Schrader, Annika, 45219 Essen (DE); Maczkiewitz, Ursula, 45219 Essen (DE); Hierath, Claudia, 45133 Essen (DE); Karacocuk, Sunay, 44625 Herne (DE); Seifert, Andreas, 46238 Bottrop (DE)
(74) Vertreter: Evonik Patent Association

(56) Entgegenhaltungen:
- WO-A1-94/26919
- WO-A2-2011/044207
- DE-A1- 19 931 310
- DE-T2- 60 225 239
- US-A1- 2006 029 657
- US-A1- 2011 065 801
- US-A1- 2011 077 302

## Beschreibung

### Gebiet der Erfindung

Gegenstand der Erfindung ist ein Verfahren zur Herstellung von Sphingolipiden, beschrieben werden zusätzlich Zusammensetzungen enthaltend Sphingolipide und weitere Komponenten sowie die Verwendung der Zusammensetzungen.

### Stand der Technik

US20110077302 offenbart ein Verfahren zur biokatalytischen Herstellung von Sphingolipiden durch Umsetzung eines Lysosphingolipids mit Carbonsäureestern dadurch gekennzeichnet, dass ein Biokatalysator eingesetzt wird, der mindestens eine Carboxylester-Hydrolase der Enzymklasse E.C. 3.1.1 aus einem Organismus des Reiches der Pilze und deren Homologe umfasst. US20110065801 offenbart ein Verfahren zur biokatalytischen Herstellung von Sphingolipiden durch Umsetzung eines Lysosphingolipids mit Glyceriden wobei ein Biokatalysator eingesetzt wird, der mindestens eine Carboxylester-Hydrolase der Enzymklasse E.C. 3.1.1 umfasst.

Die Verwendung von hohen Mengen an Lösungsmitteln und hohe Enzymkonzentrationen werden im Stand der Technik als vorteilhaft herausgestellt.

Aufgabe der vorliegenden Erfindung war die Bereitstellung eines alternativen, schonenden und industriell anwendbaren Zugangs zu Ceramiden bei dem ressourcenschonend gearbeitet werden kann.

### Beschreibung der Erfindung

Überraschenderweise wurde gefunden, dass das im Folgenden beschriebene Verfahren eine hervorragende Alternative zum Stand der Technik darstellt und überraschenderweise Zusammensetzungen bereitstellt, die vorteilhafte Eigenschaften aufweisen.

Gegenstand der vorliegenden Erfindung ist daher ein Verfahren zur Herstellung von Sphingolipiden wie in Anspruch 1 beschrieben.

Ein Vorteil des erfindungsgemäßen Verfahrens ist, dass durch den geringsten Einsatz sowohl an Lösungsmittel als auch an Enzym besonders ressourcenschonend gearbeitet werden kann. Überraschenderweise wurde zudem gefunden, dass in der lösungsmittelfreien Umsetzung, eine sehr geringe Enzymmenge zu den besten Ergebnissen führt.

Der Einsatz von Triglyceriden unter den erfindungsgemäßen Reaktionsbedingungen führt zudem zu Produktmischungen, welche sich vom Stand der Technik wie zum Beispiel der US20110065801 durch die Anwesenheit von Mono- und Diglyceriden unterscheiden und zudem besondere Effekte, insbesondere in kosmetischen Anwendungen, aufweisen.

Ein Vorteil der offenbarten Zusammensetzung ist es, dass die Zusammensetzung sich überragend in kosmetische Öle einarbeiten lassen.

Ein Vorteil der offenbarten Zusammensetzung ist es, dass die Zusammensetzung in Formulierungen überlegene sensorische Eigenschaften aufweist, die zu einem verbesserten Hautgefühl und/oder Haargefühl führen.

Ein weiterer Vorteil der offenbarten Zusammensetzung ist es, dass die Zusammensetzung in Formulierungen *in vitro* humane follikuläre dermale Papillen-Zellen (HFDPCs) zur Proliferation anregen, was *in vivo* einer Stimulation des Haarwachstums gleichkommt.

Ein weiterer Vorteil der offenbarten Zusammensetzung ist es, dass die Zusammensetzung in Formulierungen verglichen zu den Einzelkomponenten eine verbesserte Verteilbarkeit aufweist.

Ein weiterer Vorteil der offenbarten Zusammensetzung ist es, dass die Zusammensetzung in Formulierungen verglichen zu den Einzelkomponenten eine verbesserte Absorption aufweist.

Ein weiterer Vorteil der offenbarten Zusammensetzung ist es, dass die Zusammensetzung in Formulierungen verglichen zu den Einzelkomponenten eine verminderte Öligkeit aufweist.

Ein weiterer Vorteil der offenbarten Zusammensetzung ist es, dass die Zusammensetzung in Formulierungen verglichen zu den Einzelkomponenten eine verminderte Wachsigkeit aufweist.

Ein weiterer Vorteil der offenbarten Zusammensetzung ist es, dass die Zusammensetzung in Formulierungen verglichen zu den Einzelkomponenten eine verbesserte Gleitfähigkeit aufweist.

Ein weiterer Vorteil der offenbarten Zusammensetzung ist es, dass die Zusammensetzung in Formulierungen verglichen zu den Einzelkomponenten eine verminderte Klebrigkeit aufweist.

Ein weiterer Vorteil der offenbarten Zusammensetzung ist es, dass die Zusammensetzung in Formulierungen verglichen zu den Einzelkomponenten eine verbesserte Seidigkeit/Samtigkeit aufweist.

Der "pH-Wert" im Zusammenhang mit der vorliegenden Erfindung ist -wenn nicht anders angegeben - definiert als der Wert, welcher für die entsprechende Zusammensetzung bei 25 °C nach fünf Minuten Rühren mit einer gemäß ISO 4319 (1977) kalibrierten pH-Elektrode gemessen wird.

Die im Zusammenhang mit der vorliegenden Erfindung angeführten Accession-Nummern entsprechen den ProteinBank Datenbank-Einträgen des NCBI mit Datum vom 01.01.2017; in der Regel wird vorliegend die Versionsnummer des Eintrages durch ".Ziffer" wie beispielsweise ".1", kenntlich gemacht.

Alle angegebenen Prozent (%) sind, wenn nicht anders angegeben, Massenprozent.

Gegenstand der vorliegenden Erfindung ist daher ein Verfahren zur Herstellung von Sphingolipiden der allgemeinen Formel I 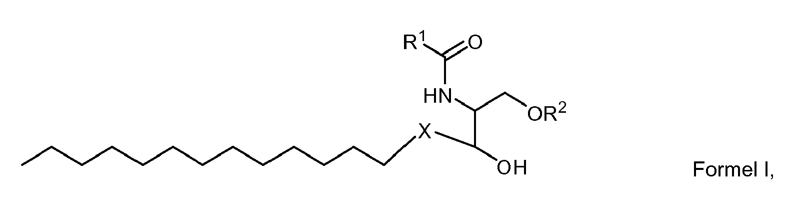 , durch Umsetzung einer ersten Komponente, einem Lysosphingolipid der allgemeinen Formel II 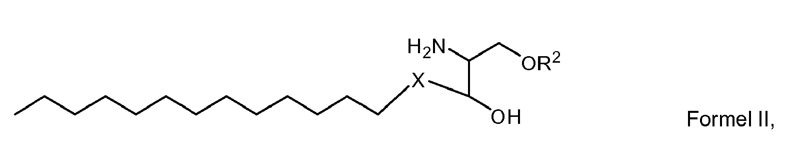
mit einer zweiten Komponente, einem Acylgruppendonor der Acylgruppe R¹CO,
wobei R¹ eine lineare oder verzweigte, gegebenenfalls eine oder mehrere Mehrfachbindungen und/oder aromatische oder heteroaromatische Ringe enthaltende, gegebenenfalls durch Sauerstoffatome oder Ester- oder Amidfunktionalitäten unterbrochene, gegebenenfalls durch mindestens eine weitere Gruppe ausgewählt aus Alkyl- Hydroxy-, Keto- oder Amingruppen substituierte Alkylkette mit 2 bis 55 Kohlenstoffatomen, bevorzugt -CH₂-Y-CH₃ mit Y = eine Kohlenstoff-Kohlenstoff-Bindung oder eine lineare oder verzweigte, gegebenenfalls eine oder mehrere Mehrfachbindungen, gegebenenfalls durch mindestens eine Hydroxygruppe substituierte Alkylenkette mit 1 bis 53, insbesondere 6 bis 32, Kohlenstoffatomen, darstellt,
R² H, Phosphocholin, Serin, Ethanolamin oder einen Zucker, bevorzugt Zucker oder H, besonders bevorzugt H, darstellt, und
X CH=CH, CH₂-CH₂ oder CH₂-HCOH, bevorzugt CH₂-CH₂ darstellt,
dadurch gekennzeichnet, dass
bezogen auf den gesamten Reaktionsansatz die erste und zweite Komponente in Summe mindestens 70 Gew.-%, bevorzugt 90 Gew.-%, insbesondere 95 Gew.-%, ausmachen, und im gesamten Reaktionsansatz nicht mehr als 600 Propyllaurat Units mindestens einer Carboxylester-Hydrolase der Enzymklasse E.C. 3.1.1 pro Gramm erster Komponente eingesetzt **werden, wobei eine Propyllaurat Unit definiert ist als die Menge Enzym, die ein µmol Propyllaurat pro Minute aus 1-Propanol und Laurinsäure synthetisiert**
und, dadurch gekennzeichnet, dass die Carboxylester-Hydrolase ausgewählt ist aus der Gruppe die Lipase aus Thermomyces lanuginosus mit accession number O59952, die Lipasen A und B mit accession number P41365 aus Candida antarctica, die Lipase aus Mucor miehei mit accession number P19515, die Lipase aus Humicola sp. mit accession number O59952, die Lipase aus Rhizomucor javanicus mit accession number S32492, die Lipase aus Rhizopus oryzae mit Accession number P61872, die Lipasen aus Candida rugosa mit accession number P20261, P32946, P32947, P3294 und P32949, die Lipase aus Rhizopus niveus mit Accession number P61871, die Lipase aus Penicillium camemberti mit accession number P25234, die Lipasen aus Aspergillus niger mit accession number ABG73613, ABG73614 10 und ABG37906 und die Lipase aus Penicillium cyclopium mit accession number P61869 umfasst, sowie jeweils deren auf Aminosäureebene mindestens 60 %, vorzugsweise mindestens 80 % bevorzugt mindestens 90 %, besonders bevorzugt mindestens 95%, 98% bzw. 99 % Homologen.

Die gemessene Aktivität der Carboxylester-Hydrolase in Propyllaurat Units wird bei der für das gegebene Enzym optimalen Temperatur gemessen, wobei unter "optimaler Temperatur" diejenige Temperatur zu verstehen ist, bei der das Enzym die höchste Aktivität aufweist. Für die Lipasen A und B mit accession number P41365 aus *Candida antarctica* liegt die optimale Temperatur beispielsweise bei 60 °C.

Als erste Komponente wird bevorzugt Sphinganin mit R² = H und X = CH₂-CH₂ eingesetzt.

Als zweite Komponente können sämtliche Acylgruppendonoren erfindungsgemäß eingesetzt werden. Solche sind beispielsweise Carbonsäureester oder Carbonsäuren selber sowie Mischungen davon.

Erfindungsgemäß bevorzugt wird der Acylgruppendonor ausgewählt aus Carbonsäureestern, bevorzugt Estern auf Basis von Alkanolen und Polyolen mit bis zu 6 C-Atomen, besonders bevorzugt mit bis zu 3 C-Atomen, ganz besonders bevorzugt Glycerinestern, eingesetzt. Erfindungsgemäß bevorzugt wird der Acylgruppendonor ausgewählt aus Acylgruppendonoren, die eine Acylgruppe ausgewählt aus der Gruppe der Acylgruppen von natürlichen Fettsäuren bereitstellen. Natürliche Fettsäuren lassen sich auf Basis natürlich vorkommender pflanzlicher oder tierischer Öle darstellen und weisen bevorzugt 6-30 Kohlenstoffatomen, insbesondere 8-22, Kohlenstoffatome auf. Natürliche Fettsäuren sind in der Regel unverzweigt und bestehen aus einer geraden Anzahl Kohlenstoffatomen. Eventuelle Doppelbindungen besitzen cis-Konfiguration. Beispiele sind: Capronsäure, Caprylsäure, Caprinsäure, Laurinsäure, Myristinsäure, Palmitinsäure, Palmitoleinsäure, Isostearinsäure, Stearinsäure, 12-Hydroxystearinsäure, Dihydroxystearinsäure, Ölsäure, Linolsäure, Linolensäure, Petrolesinsäure, Elaidinsäure, Arachinsäure, Behensäure, Erucasäure, Gadoleinsäure, Linolensäure, Eicosapentaensäure, Docosahexaensäure, Arachidonsäure,

Insbesondere erfindungsgemäß bevorzugt wird der Acylgruppendonor ausgewählt aus Triglyceriden, insbesondere natürlichen Fetten und Ölen, besonders bevorzugt ausgewählt aus der Gruppe umfassend, bevorzugt bestehend aus, Kokosfett, Palmkernöl, Olivenöl, Palmöl, Arganöl, Rizinusöl, Leinöl, Babassuöl, Rapsöl, Algenöle, Sesamöl, Sojaöl, Avocadoöl, Jojobaöl, Diestelöl, Mandelöl, Baumwollsaatöl, Sheabutter, Sonnenblumenöl, Cupuaccubutter und Öle mit einem hohen Anteil an mehrfach ungesättigten Fettsäuren (PUFAS) eingesetzt. Ebenfalls bevorzugt eingesetzt werden können Sorbitanester, Monolyceride und Digylceride mit oben beschriebenen Kettenlängenverteilungen und -modifikationen.

Es ist offenbar, dass der eingesetzte Acylgruppendonor den Rest R¹ bestimmt.

Erfindungsgemäß besonders bevorzugt wird als erste Komponente Sphinganin mit R² = H und X = CH₂-CH₂ und als zweite Komponente ein Acylgruppendonor ausgewählt aus der Gruppe umfassend, Kokosfett, Palmkernöl, Olivenöl, Palmöl, Arganöl, Rizinusöl Leinöl und Babassuöl, eingesetzt.

Die Reaktanden können zu Beginn der Umsetzung in einem molaren Verhältnis von erster zu zweiter Komponente von 1:0,11 bis 1:200.000, bevorzugt bis 1:2.000 vorliegen. Der Term "molare Verhältnis" bezieht sich hierbei auf das molare Verhältnis von Lysosphingolipid zur Anzahl der durch den Acylgruppendonor bereitgestellten Acylgruppen. Bevorzugt werden molare Mengenverhältnisse zwischen 1:0,3 und 1:200 eingesetzt. Besonders bevorzugt werden molare Mengenverhältnisse zwischen 1:1 und 1:50 eingesetzt.

In einer alternativ bevorzugten Ausführungsform liegt der Acylgruppendonor im Überschuss vor, so dass die Reaktanden zu Beginn der Umsetzung in einem molaren Verhältnis von erster zu zweiter Komponente von 1:500 bis 1:200.000, bevorzugt bis 1:2.000 vorliegen.

Da der gesamte Reaktionsansatz zum Großteil aus den Edukten, somit der ersten und zweiten Komponente besteht, kann in dem gesamten Reaktionsansatz - wenn überhaupt - nur sehr wenig Lösungsmittel enthalten sein. Aufgrund des Vorgesagten ist offenbar, dass die zweite Komponente in dem erfindungsgemäßen Verfahren nicht unter den Begriff "Lösungsmittel" fällt.

Mögliche Lösungsmittel wären beispielsweise Ketone wie beispielsweise Methylisobutylketon oder Cylclohexanon, sterisch gehinderte sekundäre Alkohole wie 2-Butyl-1-Oktanol, Methylcyclohexanole, 1-Methoxy-2-propanol, 2,3-Butandiol 2-Oktanol, Diacetonalkohol, 2-Methyl-2-butanol, und Ether wie 1,4-Dioxan, Tetrahydrofuran und Varonic APM.

Lösungsmittel sind bezogen auf den gesamten Reaktionsansatz in Summe höchstens mit weniger als 20 Gew.-%, bevorzugt weniger als 10 Gew.-%, insbesondere weniger als 5 Gew.-%, enthalten. Der Begriff "höchstens mit weniger als X Gew.-% enthalten ist" ist gleichzusetzen mit "einen Gehalt kleiner X Gew.-% beträgt".

Besonders bevorzugt wird das erfindungsgemäße Verfahren lösungsmittelfrei durchgeführt.

Bevorzugt wird das erfindungsgemäße Verfahren unter wasserfreien Bedingungen, definiert als Wassergehalt von maximal 0,100 M, bevorzugt maximal 0,010 M und besonders bevorzugt unter maximal 0,005 M, nachgewiesen nach Karl Fischer, durchgeführt.

Die im erfindungsgemäßen Verfahren eingesetzten Carboxylester-Hydrolasen sind Enzyme ausgewählt aus der Gruppe der Lipase aus *Thermomvces lanuginosus mit* accessionnumber 059952, die Lipasen A und B mit accessionnumber P41365 aus *Candida antarctica* und, die Lipase aus *Mucor miehei mit* accessionnumber *P19515,* die Lipase aus *Humicola sp.* mit accessionnumber O59952, die Lipase aus *Rhizomucor javanicus mit* accessionnumber S32492, die Lipase aus *Rhizopus oryzae* mit accessionnumber P61872, die Lipasen aus *Candida rugosa* mit accessionnumber P20261, P32946, P32947, P3294 und P32949, die Lipase aus *Rhizopus niveus mit* accessionnumber *P61871,* die Lipase aus *Penicillium camemberti mit* accessionnumber P25234, die Lipasen aus *Aspergillus nioer* mit accessionnumber ABG73613, ABG73614 und ABG37906 und die Lipase aus *Penicillium cyclopium mit* accessionnumber P61869, sowie jeweils deren auf Aminosäureebene mindestens 60 %, vorzugsweise mindestens 80 % bevorzugt mindestens 90 %. besonders bevorzugt mindestens 95%. 98% bzw. 99 % Homologen.

Die auf Aminosäureebene homologen Enzyme weisen bevorzugt verglichen zur Referenzssequenz mindestens 50 %, insbesondere mindestens 90 %, Enzymaktivität in im Zusammenhang mit der vorliegenden Erfindung definierten Propyllaurat Units auf.

Mit "Homologie auf Aminosäureebene" im Sinne der vorliegenden Erfindung ist nun und sei auch im Folgenden die "Aminosäure-Identität" verstanden, welche mit Hilfe bekannter Verfahren bestimmt werden kann. Generell werden besondere Computerprogramme mit Algorithmen unter Berücksichtigung spezieller Erfordernisse verwendet. Bevorzugte Verfahren zur Bestimmung der Identität erzeugen zunächst die größte Übereinstimmung zwischen den zu vergleichenden Sequenzen. Computer-Programme zur Bestimmung der Identität umfassen, sind jedoch nicht beschränkt auf, das GCG- Programmpaket, einschließlich
- GAP (Deveroy, J. et al., Nucleic Acid Research 12 (1984), Seite 387, Genetics Computer Group University of Wisconsin, Medicine (WI), und
- BLASTP, BLASTN und FASTA (Altschul, S. et al., Journal of Molecular Biology 215 (1990), Seiten 403-410. Das BLAST-Programm kann erhalten werden vom National Center For Biotechnology Information (NCBI) und aus weiteren Quellen (BLAST Handbuch, Altschul S. et al., NCBI NLM NIH Bethesda ND 22894; Altschul S. et al., vorstehend).

Der Fachmann ist sich bewusst, dass verschiedene Computerprogramme für die Kalkulation der Ähnlichkeit bzw. Identität zwischen zwei Nukleotid- oder Aminosäure-Sequenzen zur Verfügung stehen. So kann die prozentuale Identität zwischen zwei Aminosäure-Sequenzen z.B. durch den Needleman und Wunsch (J. Mol. Biol. (48): 444-453 (1970)) Algorithmus bestimmt werden, der in das GAP Programm im GCG Software-Paket (erhältlich über http://www.gcg.com) integriert wurde, und zwar entweder unter Verwendung einer Blossom 62-Matrix oder einer PAM250-Matrix, einer gap weight von 16, 14, 12, 10, 8, 6, oder 4 und einer length weight von 1, 2, 3, 4, 5, oder 6. Der Fachmann wird anerkennen, dass die Verwendung unterschiedlicher Parameter zu leicht unterschiedlichen Ergebnissen führen wird, dass aber die prozentuale Identität zwischen zwei Aminosäure-Sequenzen insgesamt nicht signifikant unterschiedlich sein wird. Üblicherweise wird die Blossom 62-Matrix unter Anwendung der Voreinstellungen (gap weight: 12, length weight: 1) genutzt.

Eine Identität von 60 % gemäß dem vorstehenden Algorithmus bedeutet im Zusammenhang mit der vorliegenden Erfindung 60 % Homologie. Das gleiche gilt für höhere Identitäten.

Kommerzielle Beispiele und ebenfalls bevorzugt eingesetzte Carboxylester-Hydrolasen in erfindungsgemäßem Verfahren sind die Handelsprodukte Lipozyme TL IM, Novozym 435, Lipozyme IM 20, Lipase SP382, Lipase SP525, Lipase SP523, (alles Handelsprodukte der Firma Novozymes A/S, Bagsvaerd, Dänemark), Chirazyme L2, Chirazyme L5, Chirazyme L8, Chirazyme L9 (alles Handelprodukte der Firma Roche Molecular Biochemicals, Mannheim, Deutschland), CALB Immo Plus TM der Firma Purolite, sowie Lipase M "Amano", Lipase F-AP 15 "Amano", Lipase AY "Amano", Lipase N "Amano", Lipase R "Amano", Lipase A "Amano", Lipase D "Amano", Lipase G "Amano" (alles Handelsprodukte der Firma Amano, Japan),

Das erfindungsgemäße Verfahren wird bevorzugt bei Reaktionstemperaturen im Bereich zwischen 20 °C und 160 °C, bevorzugt 35 und 130 insbesondere zwischen 65 °C und 110 °C durchgeführt.

Das erfindungsgemäße Verfahren wird bevorzugt bei einem Druck von kleiner 1 bar, bevorzugt kleiner 0,5 bar und besonders bevorzugt kleiner 0,05 bar durchgeführt.

In einer alternativen bevorzugten Ausführungsform wird das erfindungsgemäße Verfahren bei einem Druck von größer 1 bar, bevorzugt in einem Bereich von 2 bar bis 10 bar, durchgeführt. In diesem Zusammenhang ist es bevorzugt, dass der Reaktionsansatz mit einem Inertgas versehen wird; diese sind bevorzugt ausgewählt aus der Gruppe umfassend, bevorzugt bestehend aus, Stickstoff und Argon.

Folgende Abbildungen sind Bestandteil der Beispiele:
Abbildung 1: Überlebenswahrscheinlichkeit der Einzelhaare
Abbildung 2: Überlebenswahrscheinlichkeit von alkalisch geglätteten Haaren nach Behandlung mit Ceramiden
Abbildung 3: Überlebenswahrscheinlichkeit ethnisches Haar

### Beispiele:

### Beispiel 1: Bestimmung der spezifischen Aktivität des eingesetzten Enzyms in PLU

Zur Ermittlung der Enzymaktivität in PLU (Propyl Laurate Unit) werden 1-Propanol und Laurinsäure in äquimolarem Verhältnis bei 60°C homogen gemischt. Mit Enzymzugabe wird die Reaktion gestartet und die Reaktionszeit gestoppt. In Abständen werden Proben aus der Reaktionsmischung entnommen und der Gehalt an umgesetzter Laurinsäure mittels Titration mit Kaliumhydroxid-Lösung bestimmt. Die Enzymaktivität in PLU ergibt sich aus der Geschwindigkeit, in der 1 g des betrachteten Enzyms 1 µmol Propyllaurat pro min bei 60°C synthetisiert, vgl. hierzu auch US20070087418, insbesondere [0185].

Der Hersteller Novozymes beispielsweise gibt in seinem Produktdatenblatt von Novozym 435 10000 PLU/g an.

### Beispiel 2: Umsetzung von Sphinganin mit Olivenöl, olivenöl-basiertes Ceramid NG

12g Olivenöl werden mit 8 g Sphinganin in 2,2 g 2-Methyl-2-butanol gelöst Die Mischung wird auf 100 °C erwärmt, mit 0,05 bar Stickstoff überlagert und gerührt. Nach Erreichen der Reaktionstemperatur werden 120 PLU Novozym 435 hinzugegeben und weiter gerührt. Nach 24 h wurde Novozym 435 abfiltriert. Das so erhaltene Reaktionsgemisch erreichte bezogen auf Sphinganin einen Umsatz von >98,5 %. Das Lösungsmittel wird nach der Umsetzung durch Destillation entfernt und das erhaltene Produktgemisch in Anwendungstests eingesetzt.

### Beispiel 3: Hochölhaltige Zusammensetzung

10 g einer Zusammensetzung aus Beispiel 2 werden in 90 g einer 1:1 (Gewicht) Mischung Olivenöl und Rizinusöl gelöst: Es entsteht eine hochölhaltige Zusammensetzung enthaltend 10% einer Mischung aus olivenöl-basiertem Ceramid NG, Glycerin, Glyceriden und Sphinganin mit 45% Olivenöl und 45% Rizinusöl

### Beispiel 4: Umsetzung von Sphinganin mit Rizinusöl

25 g Rizinusöl werden mit 16 g Sphinganin gemischt. Die Mischung wird auf 100°C erwärmt, mit 0,05 bar Stickstoff überlagert und gerührt. Nach Erreichen der Reaktionstemperatur werden 80 PLU Novozym 435 (bezogen auf Sphinganin) hinzugegeben und weiter gerührt. Nach 24 h wurde Novozym 435 abfiltriert. Das so erhaltene Reaktionsgemisch wurde mit 60 ml Rizinusöl abgemischt. Bezogen auf Sphinganin erreichte die Umsetzung einen Umsatz von >98,5 %.

### Beispiel 5: Einfluss der Lösungsmittelmenge

12 g Olivenöl werden mit 8 g Sphinganin gemischt und mit der in der Tabelle unten angegebenen Menge an 2-Methyl-2-butanol versetzt. Die Mischung wird auf 80°C erwärmt, mit 0,05 bar Stickstoff überlagert und gerührt. Nach Erreichen der Reaktionstemperatur wird die angegebenen Menge Novozym 435 hinzugegeben und weiter gerührt. Nach 2 h wurde jeweils eine Probe entnommen und mittels GC auf den Restgehalt Sphinganin untersucht. Die Ergebnisse zeigen, dass bei abnehmender Menge Lösungsmittel bessere Umsätze erreicht werden können, wenn weniger Enzym eingesetzt wird. Die durchgeführte Negativkontrolle ohne Zusatz von Lösungsmittel und Enzym ergab einen Umsatz ungleich null.

| Enzymmenge in PLU/g Sphinganin | Lösungsmittel 2-Methyl-2-butanol in % | Umsatz in % |
|---|---|---|
| 120 | 10 | 26 |
| 25 | 10 | 24 |
| 120 | 1 | 27 |
| 25 | 0 | 28 |

### Beispiel 6: Weitere Synthesebeispiele

Die allgemeine Durchführung erfolgte wie in Beispiel 2 beschrieben ohne den Einsatz von Lösungsmitteln.

| Name | Acyldonor | Menge Acyldonor in g | Menge Sphinganin in g | Enzymmenge in in PLU/g Sphinganin | Temperatur °C | Umsatz nach 24 h in % bezogen auf Sphinganin |
|---|---|---|---|---|---|---|
| TG1 | Olivenöl | 448 | 41 | 200 | 95 | >98 |
| TG2 | Olivenöl | 29 | 41 | 200 | 100 | >98 |
| | Rizinusöl | 31 | | | | |
| TG3 | Rizinusöl | 24 | 16 | 120 | 85 | >98 |
| TG4a | Rapsöl | 73 | 25 | 100 | 90 | >95 |
| TG4b | Rapsöl | 88 | 10 | 100 | 90 | >95 |
| TG5 | Sesamöl | 10 | 7 | 150 | 90 | >95 |
| TG6 | Palmkernöl | 56 | 25 | 100 | 90 | >95 |
| TG7a | Palmöl | 70 | 25 | 100 | 90 | >95 |
| TG7b | Palmöl | 84 | 10 | 100 | 90 | >95 |
| TG8 | Mandelöl | 10 | 7 | 100 | 90 | >90 |
| TG9 | Sorbitanmonooleat | 60 | 50 | 100 | 90 | >70 |
| TG10 | Sorbitanmonolaurat | 58 | 50 | 100 | 90 | >70 |
| TG11 | Sorbitantrioleat | 83 | 50 | 100 | 90 | >70 |
| TG12 | Sojaöl | 87 | 10 | 100 | 90 | >90 |
| TG13 | Glycoldistearat | 50 | 25 | 100 | 90 | >70 |
| TG14 | Cetyltriglycerid | 83 | 50 | 100 | 90 | >90 |

### Beispiel 7: Anwendungsdaten Haut: Einfluss auf Hautfeuchte, Trockenheit gemessen durch Schuppigkeit, Hautbarriere gemessen an allgemeinem Hautzustand in vivo.

Zur Bestimmung der Hautpflegeeigenschaften der hochölhaltigen Zusammensetzung aus Beispiel 3 wurde eine *in-vivo* Studie durchgeführt. Der Ablauf der Studie war wie folgt: Die Probanden erhielten jeweils 2 Testformulierungen, die für eine Dauer von 2 Wochen zweimal täglich auf die Innenseite der Unterarme aufgetragen werden mussten. Vor Beginn der Anwendung sowie nach 1 und nach 2 Wochen wurde mit einer speziellen Kamera (Visioscan VC 98, Courage & Khazaka, Köln) ein Schwarz-Weiß-Bild der Haut aufgenommen. Anhand dieser Bilder berechnete die Software der Kamera die Hautschuppigkeit und die Hautrauhigkeit. Die Werte errechnen sich aus der Graustufenverteilung der Bilder, daher besitzen die Ergebnisse keine Einheit. Außerdem erhielten die Probanden einen Fragebogen zur Beurteilung der pflegenden Eigenschaften der Testformulierungen. Insgesamt nahmen 25 Probanden an der Studie teil.

| Inhaltsstoff | Vehikel | Formulierung mit Wirkstoff |
|---|---|---|
| Glyceryl Stearate Citrate | 2,00% | 2,00% |
| Cetearyl Alcohol | 1,00% | 1,00% |
| Caprylic/Capric Triglyceride | 4,80% | 4,80% |
| C12-15 Alkyl Benzoate | 4,00% | 4,00% |
| Beispiel 3 (% bezogen auf Sphingolipid) | | 1,00% |
| Carbomer | 0,20% | 0,20% |
| Ethylhexylglycerin, Phenoxyethanol | 1,00% | 1,00% |
| Natriumhydroxid (10% in Wasser) | 0,70% | 0,70% |
| Wasser | ad 100 % | ad 100 % |

| | Abnahme der Hautrauhigkeit | | Abnahme der Hautschuppigkeit | |
|---|---|---|---|---|
| | Vehikel | Beispiel 3 | Vehikel | Beispiel 3 |
| nach 1 Woche | -1,58 | -11,08 | -0,0271 | -0,1292 |
| nach 2 Wochen | -5,61 | -15,04 | -0,0761 | -0,1313 |

| | Das Produkt reduziert die Schuppigkeit der trockenen Haut | | Das Produkt versorgt die Haut ausreichend mit Feuchtigkeit | | Mein Hautzustand hat sich verbessert | |
|---|---|---|---|---|---|---|
| Das Produkt reduziert die Schuppigkeit der trockenen Haut | Vehikel | Beispiel 3 | Vehikel | Beispiel 3 | Vehikel | Beispiel 3 |
| Stimme voll zu | 5.3 | 13.6 | 22.7 | 30.4 | 4.5 | 4.5 |
| stimme zu | 31.6 | 40.9 | 45.5 | 52.2 | 22.7 | 54.5 |
| Stimme weder zu noch nicht zu | 57.9 | 40.9 | 31.8 | 17.4 | 72.7 | 40.9 |
| stimme eher nicht zu | 5.3 | 4.5 | 0.0 | 0.0 | 0.0 | 0.0 |
| stimme gar nicht zu | 0.0 | 0.0 | 0.0 | 0.0 | 0.0 | 0.0 |

| | | | | | | |
|---|---|---|---|---|---|---|
| Ergebnisse der Probandenbefragung. | | | | | | |

Sowohl die Hautschuppigkeit als auch die Hautrauhigkeit wurden nach Anwendung der Formulierung mit der hochölhaltigen Zusammensetzung aus Beispiel 3 stärker reduziert als nach Anwendung der Vehikel-Formulierung. Diese Ergebnisse wurden auch durch die Probanden bestätigt. Hier sieht man eine deutlich höhere Zustimmung zu den Aussagen "Die Formulierung reduziert die Schuppigkeit der trockenen Haut", "Das Produkt versorgt die Haut ausreichend mit Feuchtigkeit" und "Mein Hautzustand hat sich verbessert" mit der Testformulierung, die das Produkt aus Beispiel 3 enthält, als bei der Vehikel-Formulierung.

### Beispiel 8: Anwendungsdaten Haare: Reparatur von UV-geschädigten Haaren.

Im folgenden Beispiel soll die Reparatur-Wirkung eines Testkonditionierers, der das Produkt aus Beispiel 2 enthält, mit der Reparatur-Wirkung von Ceramide NP verglichen werden. Kaukasische, ungeschädigte Haare wurden 24h UV bestrahlt. Hierfür wurde eine UV-Kammer benutzt (Dr. Höhnle, Sol 2). Die Haarsträhnen wurden mit einer Leistung von 910 W/m2 bestrahlt. Anschließend wurden die Haare folgendermaßen behandelt:
1. Waschen mit einem Testshampoo bestehend aus Natriumlaurylethersulfat und Cocamidopropylbetain.
2. Applikation des Testkonditionierers, die Einwirkzeit betrug 5 min. Anschließend wurden die Haarsträhnen 1 min unter fließendem Leitungswasser mit einer Temperatur von 37 °C ausgespült.
3. Trocknen der Haarsträhnen für 3 min mit einem Fön.

Diese drei Behandlungsschritte wurden insgesamt 5x durchgeführt.

### Testformulierungen:

### Shampoo

| Inhaltsstoff | Konzentration |
|---|---|
| Sodium Laureth Sulfate (28%) | 32,10% |
| Wasser | 60,50% |
| Cocomidopropyl Betaine (47.5%) | 6,40% |
| Phenoxyethanol, Ethylhexylglycerin | 1,00% |

| .Inhaltsstoff | Vehikel | Beispiel 2 | Ceramide NP |
|---|---|---|---|
| Ceteareth-25 | 0,50% | 0,50% | 0,50% |
| Cetearyl Alcohol | 4,00% | 4,00% | 4,00% |
| Cetrimonium Chloride | 1,50% | 1,50% | 1,50% |
| Ceramidmischung aus Beispiel 2 (% bezogen auf Sphingolipid) | | 0,10% | |
| Ceramide NP | | | 0,1% |
| Wasser | ad 100 % | ad 100 % | ad 100 % |
| Phenoxyethanol, Ethylhexylglycerin | 1,00% | 1,00% | 1,00% |
| Milchsäure (10%) | pH 4,0-4,5 | pH 4,0-4,5 | pH 4,0-4,5 |

| | | | |
|---|---|---|---|
| Testkonditionierer für die Reparatur von UV-geschädigten Haaren. | | | |

Zur Quantifizierung der Reparaturwirkung wurde eine Haarbruchmessung durchgeführt (Cyclic Tester, Diastron limited, UK). Bei dieser Messung werden Einzelhaare solange mit einer konstanten Kraft gedehnt, bis sie reißen. Die Anzahl der überlebten Dehnungszyklen wird aufgezeichnet und dient zur Berechnung der Überlebenswahrscheinlichkeit. Für jede Messung wurden ca. 50 Einzelhaare verwendet.

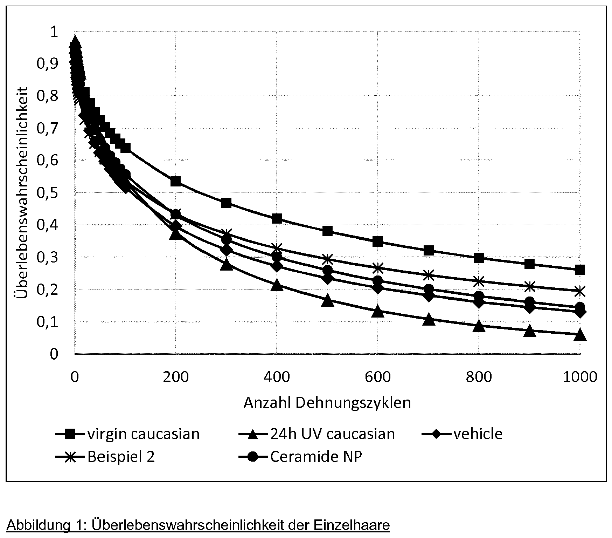

Die Abbildung 1 zeigt, dass die Überlebenswahrscheinlichkeit von UV-bestrahlten Haaren im Haarbruchtest deutlich geringer ist als bei nicht-bestrahlten Haaren (virgin caucasian). Durch die Behandlung mit den Ceramid-haltigen Testformulierungen wurde die Überlebenswahrscheinlichkeit wieder erhöht. Dabei erkennt man, dass die Formulierung, die das Produkt aus Beispiel 2 enthält, zu einer höheren Überlebenswahrscheinlichkeit führt als das zum Vergleich getestete Ceramide NP.

### Beispiel 9: Anwendungsdaten Haare: Schutz vor Proteindegradierung.

Ethnische Haare wurden chemisch geglättet. Hierfür wurde eine Standardformulierung auf Basis von Guanidiniumcarbonat und Kalziumhydroxid verwendet mit einem pH-Wert von ca. 12. Anschließend wurden die Haare folgendermaßen behandelt:
1. Waschen mit einem Shampoo bestehend aus Natriumlaurylethersulfat und Cocamidopropylbetain.
2. Applikation des Testkonditionierers, die Einwirkzeit betrug 5 min. Anschließend wurden die Haarsträhnen 1 min unter fließendem Leitungswasser mit einer Temperatur von 37 °C ausgespült.
3. Trocknen der Haarsträhnen für 3 min mit einem Fön.

Diese drei Behandlungsschritte wurden insgesamt 5x durchgeführt.

### Testformulierung:

| Inhaltsstoff | Konzentration |
|---|---|
| Sodium Laureth Sulfate (28%) | 32,10% |
| Wasser | 60,50% |
| Cocomidopropyl Betaine (47.5%) | 6,40% |
| Phenoxyethanol, Ethylhexylglycerin | 1,00% |

### Shampoo

| Inhaltsstoff | Vehikel | Beispiel 2 | Formulierung mit Ceramide II |
|---|---|---|---|
| Ceteareth-25 | 0,50% | 0,50% | 0,50% |
| Cetearyl Alcohol | 4,00% | 4,00% | 4,00% |
| Cetrimonium Chloride | 1,50% | 1,50% | 1,50% |
| Ceramidmischung aus Beispiel 2 (% bezogen auf Sphingolipid) | | 0,10% | |
| Ceramide II (Sederma) | | | 0,10% |
| Wasser | ad 100 % | ad 100 % | ad 100 % |
| Phenoxyethanol, Ethylhexylglycerin | 1,00% | 1,00% | 1,00% |
| Milchsäure (10%) | pH 4,0-4,5 | pH 4,0-4,5 | pH 4,0-4,5 |

| | | | |
|---|---|---|---|
| Testkonditionierer | | | |

Zur Quantifizierung der Reparaturwirkung wurde eine Haarbruchmessung durchgeführt (Cyclic Tester, Diastron limited, UK). Bei dieser Messung werden Einzelhaare solange mit einer konstanten Kraft gedehnt, bis sie reißen. Die Anzahl der überlebten Dehnungszyklen wird aufgezeichnet und dient zur Berechnung der Überlebenswahrscheinlichkeit. Für jede Messung wurden ca. 50 Einzelhaare verwendet.

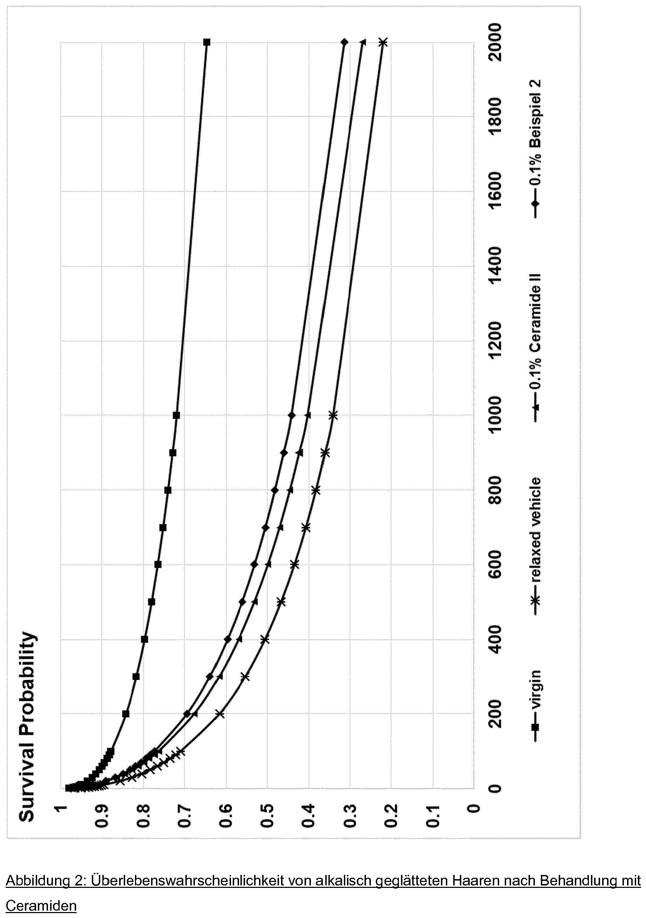

Die Abbildung 2 zeigt, dass die Überlebenswahrscheinlichkeit von alkalisch geglätteten Haaren im Haarbruchtest deutlich geringer ist als bei nicht-geglätteten Haaren (virgin). Durch die Behandlung mit den Ceramid-haltigen Testformulierungen wurde die Überlebenswahrscheinlichkeit wieder erhöht. Überraschenderweise wurde gefunden, dass die erfindungsgemäßen Zusammensetzungen (Beispiel 2) zu einer höheren Überlebenswahrscheinlichkeit führen als das für Haarpflegeprodukte handelsübliche Ceramide II (reines Sphinganine C18).

### Beispiel 10: Anwendungsdaten Haare: Reparatur des Haares nach saurem Glätten.

Ethnische Haare wurden sauer geglättet und mit einem Conditioner nachbehandelt, welcher eine der beanspruchten Zusammensetzungen mit erhöhtem Triglyceridanteil, bestehend aus: 10% olivenöl-basiertem Ceramid (Beispiel 2) enthielt. Zum Vergleich wurden das Verfahren mit einer entsprechenden Menge an reinem Ceramid NG bzw. reinem Olivenöl durchgeführt. Gemessen wurden der Reparatureffekt und die Stärkung des CMC-Komplexes mittels *Hair Fatigue alpha.*

Ethnische Haare wurden chemisch geglättet. Hierfür wurde eine saure Standardformulierung mit einem pH-Wert von ca. 1-2 in Kombination mit der Anwendung eines Glätteisens verwendet. Anschließend wurden die Haare folgendermaßen behandelt:
1. Waschen mit einem Shampoo bestehend aus Natriumlaurylethersulfat und Cocamidopropylbetain.
2. Applikation des Testkonditionierers, die Einwirkzeit betrug 5 min. Anschließend wurden die Haarsträhnen 1 min unter fließendem Leitungswasser mit einer Temperatur von 37 °C ausgespült.
3. Trocknen der Haarsträhnen für 3 min mit einem Fön.

Diese drei Behandlungsschritte wurden insgesamt 5x durchgeführt.

Der Testkonditionierer enthielt entweder keinen Wirkstoff (Vehikel, 1% Olivenöl, 0,1% Ceramide NG oder die beanspruchten Zusammensetzungen mit erhöhtem Triglyceridanteil.

| Inhaltsstoff | Konzentration |
|---|---|
| Sodium Laureth Sulfate (28%) | 32,10% |
| Wasser | 60,50% |
| Cocomidopropyl Betaine (47.5%) | 6,40% |
| Phenoxyethanol, Ethylhexylglycerin | 1,00% |

| | |
|---|---|
| Shampoo | |

| Inhaltsstoff | Vehikel | Formulierung mit Beispiel 3 | Formulierung Olivenöl | Formulierung mit Ceramide NG |
|---|---|---|---|---|
| Ceteareth-25 | 0,50% | 0,50% | 0,50% | 0,50% |
| Cetearyl Alcohol | 4,00% | 4,00% | 4,00% | 4,00% |
| Cetrimonium Chloride | 1,50% | 1,50% | 1,50% | 1,50% |
| Ceramidmischung aus Beispiel 3 (% bezogen auf Sphingolipid) | | 1,00% | | |
| Olivenöl | | | 1,00% | |
| Ceramide NG | | | | 0,10% |
| Wasser | ad 100 % | ad 100 % | ad 100 % | ad 100 % |
| Phenoxyethanol, Ethylhexylglycerin | 1,00% | 1,00% | 1,00% | 1,00% |
| Milchsäure (10%) | pH 4,0-4,5 | pH 4,0-4,5 | pH 4,0-4,5 | pH 4,0-4,5 |

| | | | | |
|---|---|---|---|---|
| Testkonditionierer | | | | |

Zur Quantifizierung der Reparaturwirkung wurde eine Haarbruchmessung durchgeführt (Cyclic Tester, Diastron limited, UK). Bei dieser Messung werden Einzelhaare solange mit einer konstanten Kraft gedehnt, bis sie reißen. Die Anzahl der überlebten Dehnungszyklen wird aufgezeichnet und dient zur Berechnung der Überlebenswahrscheinlichkeit. Für jede Messung wurden ca. 50 Einzelhaare verwendet.

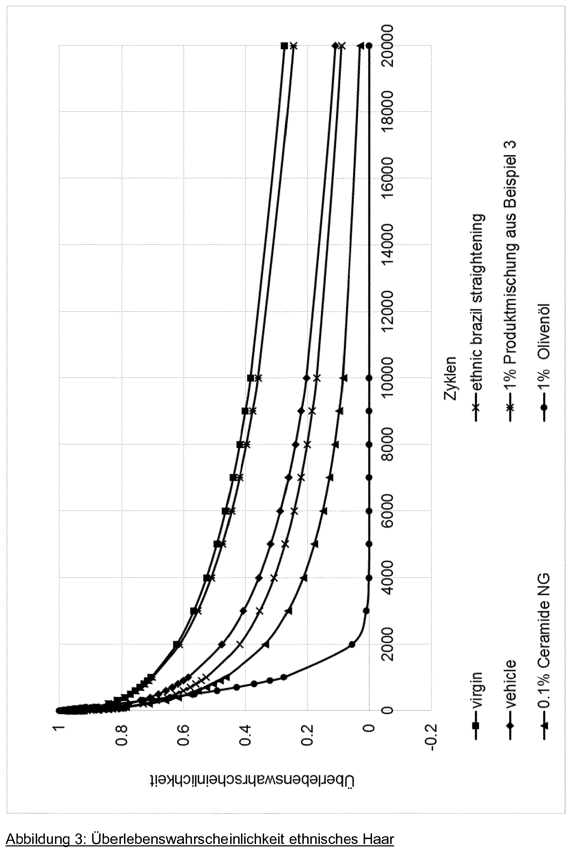

Überraschenderweise wurde - wie Abbildung 3 gezeigt - gefunden, dass ausschließlich die erfindungsgemäße Zusammensetzung einen entsprechenden Reparatureffekt erzielen konnte, wohingegen Ceramid NG und Olivenöl keinen Reparatureffekt erzielen konnten.

### Beispiel 11: Anwendungsdaten Formulierbarkeit: Kristallfreies Einarbeiten in kosmetische Öle.

Die geringe Löslichkeit und große Neigung zu Rekristallisation von Sphingolipiden erschweren seit jeher die stabile Einarbeitung in kosmetischen Formulierungen.

Überraschenderweise wurde gefunden, dass der Einsatz der erfindungsgemäßen hochölhaltigen Zusammensetzungen mit erhöhtem Triglyceridanteil zu einer deutlich vereinfachten Einarbeitbarkeit der enthaltenen Sphingolipide in kosmetische Öle führt, als bei Sphingolipiden des Standes der Technik.

Es wurden hierfür die Löslichkeitstemperaturen der Sphingolipide ermittelt:
Eine Endkonzentration von 0,1% Ceramid in kosmetischem Öl wurde gewählt. Die Ceramidzusammensetzungen wurden im Becherglas auf einer Heizplatte in den kosmetischen Ölen langsam erwärmt und gerührt. Die Löslichkeitstemperatur war erreicht, sobald sich eine klare Lösung ergab. Es wurde anschließend bei der ermittelten Löslichkeitstemperatur 1 Stunde gerührt. Nach Abkühlen auf Raumtemperatur wurde die Formulierung untersucht.

| Emollient | Polarität | Beispiel 3 | | reines Ceramide NP | |
|---|---|---|---|---|---|
| | | Löslichkeitstemperatur | Aussehen bei RT | Löslichkeitstemperatur | Aussehen bei RT |
| Octyldodecanol | polar | 40°C | klar | 70°C | Kristalle |
| TEGOSOFT APM | polar | 35°C | klar | 65°C | Kristalle |
| TEGOSOFT CT | polar - medium polar | 35°C | klar | 95°C | Kristalle |
| TEGOSOFT M | medium polar | 45°C | klar | 90°C | Kristalle |
| TEGOSOFT OS | non-polar | 50°C | klar | 100°C | Kristalle |
| Mineral Oil | non-polar | 60°C | trüb | 110°C | Kristalle |

Die Daten zeigen eine deutlich verbesserte Einarbeitbarkeit der erfindungsgemäßen hochölhaltigen Zusammensetzungen in kosmetische Öle verglichen zu reinen Ceramiden.

## Patentansprüche

1. Verfahren zur Herstellung von Sphingolipiden der allgemeinen Formel I 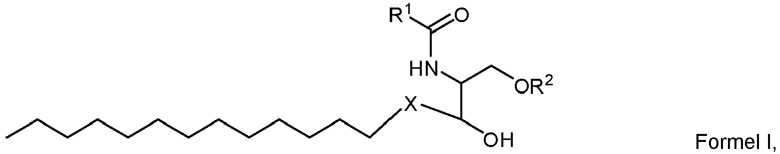 durch Umsetzung einer ersten Komponente, einem Lysosphingolipid der allgemeinen 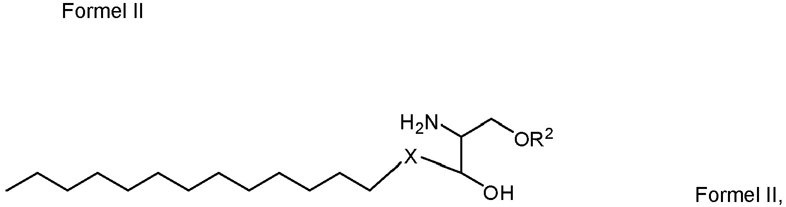 mit einer zweiten Komponente, einem Acylgruppendonor der Acylgruppe R¹CO,
wobei
R¹ eine lineare oder verzweigte, gegebenenfalls eine oder mehrere Mehrfachbindungen und/oder aromatische oder heteroaromatische Ringe enthaltende, gegebenenfalls durch Sauerstoffatome oder Ester- oder Amidfunktionalitäten unterbrochene, gegebenenfalls durch mindestens eine weitere Gruppe ausgewählt aus AlkylHydroxy-, Keto- oder Amingruppen substituierte Alkylkette mit 2 bis 55 Kohlenstoffatomen darstellt,
R² H, Phosphocholin, Serin, Ethanolamin oder einen Zucker, bevorzugt Zucker oder H, besonders bevorzugt H, darstellt, und
X CH=CH, CH₂-CH₂ oder CH₂-HCOH, bevorzugt CH₂-CH₂, darstellt,
**dadurch gekennzeichnet, dass**
bezogen auf den gesamten Reaktionsansatz die erste und zweite Komponente in Summe mindestens 70 Gew.-%, bevorzugt 90 Gew.-%, insbesondere 95 Gew.-%, ausmachen, und im gesamten Reaktionsansatz nicht mehr als 600 Propyllaurat Units mindestens einer Carboxylester-Hydrolase der Enzymklasse E.C. 3.1.1 pro Gramm erster Komponente eingesetzt werden, wobei eine Propyllaurat Unit definiert ist als die Menge Enzym, die ein µmol Propyllaurat pro Minute aus 1-Propanol und Laurinsäure synthetisiert und, **dadurch gekennzeichnet, dass**
die Carboxylester-Hydrolase ausgewählt ist aus der Gruppe die Lipase aus *Thermomyces lanuginosus mit* accession number *O59952*, die Lipasen A und B mit accession number P41365 aus *Candida antarctica* und, die Lipase aus *Mucor miehei mit* accession number P19515, die Lipase aus *Humicola sp.* mit accession number O59952, die Lipase aus *Rhizomucor javanicus mit* accession number S32492, die Lipase aus *Rhizopus oryzae* mit accession number P61872, die Lipasen aus *Candida rugosa* mit accession number P20261, P32946, P32947, P3294 und P32949, die Lipase aus *Rhizopus niveus mit* accession number P61871, die Lipase aus *Penicillium camemberti mit* accession number P25234, die Lipasen aus *Aspergillus niger mit* accession number ABG73613, ABG73614 und ABG37906 und die Lipase aus *Penicillium cyclopium mit* accession number P61869, umfasst, sowie jeweils deren auf Aminosäureebene mindestens 60 %, vorzugsweise mindestens 80 % bevorzugt mindestens 90 %, besonders bevorzugt mindestens 95%, 98% bzw. 99 % Homologen, wobei die Accession-Nummern den ProteinBank Datenbank-Einträgen des NCBI mit Datum vom 01.01.2017 entsprechen.

2. Verfahren nach Anspruch 1, **dadurch gekennzeichnet, dass** als erste Komponente Sphinganin mit R² = H und X = CH₂-CH₂ eingesetzt wird.

3. Verfahren nach Anspruch 1 oder 2, **dadurch gekennzeichnet, dass** die zweite Komponente ausgewählt ist aus Acylgruppendonoren, die eine Acylgruppe ausgewählt aus der Gruppe der Acylgruppen von natürlichen Fettsäuren bereitstellen.

4. Verfahren nach mindestens einem der vorherigen Ansprüche, **dadurch gekennzeichnet, dass** die zweite Komponente ausgewählt ist aus Estern auf Basis von Alkanolen und Polyolen mit bis zu 6 C-Atomen, besonders bevorzugt mit bis zu 3 C-Atomen, ganz besonders bevorzugt aus Glycerinestern.

5. Verfahren nach mindestens einem der vorherigen Ansprüche, **dadurch gekennzeichnet, dass** als erste Komponente Sphinganin mit R² = H und X = CH₂-CH₂ und als zweite Komponente ein Acylgruppendonor ausgewählt aus der Gruppe umfassend Kokosfett, Palmkernöl, Olivenöl, Palmöl, Arganöl, Rizinusöl, Leinöl und Babassuöl eingesetzt wird.

6. Verfahren nach mindestens einem der vorherigen Ansprüche, **dadurch gekennzeichnet, dass** die Reaktanden zu Beginn der Umsetzung in einem molaren Verhältnis von erster zu zweiter Komponente von 1:0,11 bis 1:2000 vorliegen.

7. Verfahren nach mindestens einem der vorherigen Ansprüche, **dadurch gekennzeichnet, dass** bezogen auf den gesamten Reaktionsansatz in Summe ein Lösungsmittel höchstens mit weniger als 20 Gew.-%, bevorzugt weniger als 10 Gew.-%, insbesondere weniger als 5 Gew.-%, enthalten ist.

8. Verfahren nach mindestens einem der vorherigen Ansprüche, **dadurch gekennzeichnet, dass** es unter wasserfreien Bedingungen durchgeführt wird.

## Claims

1. Process for preparing sphingolipids of the general formula I 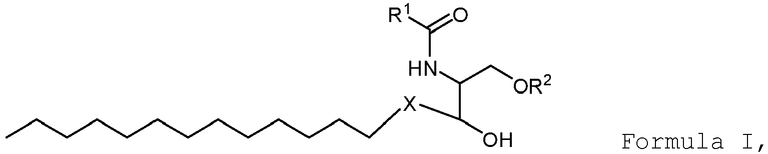 by reacting a first component, a lysosphingolipid of the general formula II 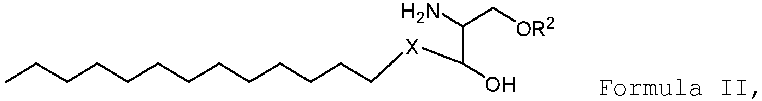 with a second component, an acyl group donor of the acyl group R¹CO,
where
R¹ represents a linear or branched alkyl chain having 2 to 55 carbon atoms that optionally contains one or more multiple bonds and/or aromatic or heteroaromatic rings, is optionally interrupted by oxygen atoms or ester or amide functionalities and is optionally substituted by at least one further group selected from alkyl, hydroxyl, keto or amine groups,
R² represents H, phosphocholine, serine, ethanolamine or a sugar, preferably sugar or H, more preferably H, and
X represents CH=CH, CH₂-CH₂ or CH₂-HCOH, preferably CH₂-CH₂,
**characterized in that**
the first and second components, based on the overall reaction mixture, account for a total of at least 70% by weight, preferably 90% by weight, especially 95% by weight,
and not more than 600 propyl laurate units of at least one carboxylic ester hydrolase from the E.C. 3.1.1 enzyme class per gram of first component are used in the entire reaction mixture, where one propyl laurate unit is defined as the amount of enzyme that synthesizes one µmol of propyl laurate per minute from 1-propanol and lauric acid, and **characterized in that**
the carboxylic ester hydrolase is selected from the group comprising the lipase from *Thermomyces lanuginosus* with accession number 059952, lipases A and B with accession number P41365 from *Candida antarctica* and the lipase from *Mucor miehei* with accession number *P19515,* the lipase from *Humicola sp.* with accession number O59952, the lipase from *Rhizomucor javanicus* with accession number S32492, the lipase from *Rhizopus oryzae* with accession number P61872, the lipases from *Candida rugosa* with accession number P20261, P32946, P32947, P3294 and P32949, the lipase from *Rhizopus niveus* with accession number *P61871,* the lipase from *Penicillium camemberti* with accession number P25234, the lipases from *Aspergillus niger* with accession number ABG73613, ABG73614 and ABG37906, and the lipase from *Penicillium cyclopium* with accession number P61869, and their respective at least 60%, preferably at least 80%, more preferably at least 90%, especially preferably at least 95%, 98% or 99%, homologues at the amino acid level, where the accession numbers correspond to the NCBI ProteinBank database entries as at 01.01.2017.

2. Process according to Claim 1, **characterized in that** the first component used is sphinganine with R² = H and X = CH₂-CH₂.

3. Process according to Claim 1 or 2, **characterized in that** the second component is selected from acyl group donors that provide an acyl group selected from the group of acyl groups of natural fatty acids.

4. Process according to at least one of the preceding claims, **characterized in that** the second component is selected from esters based on alkanols and polyols having up to 6 carbon atoms, more preferably having up to 3 carbon atoms, most preferably from glycerol esters.

5. Process according to at least one of the preceding claims, **characterized in that** the first component used is sphinganine with R² = H and X = CH₂-CH₂, and the second component used is an acyl group donor selected from the group comprising coconut fat, palm kernel oil, olive oil, palm oil, argan oil, castor oil, linseed oil and babassu oil.

6. Process according to at least one of the preceding claims, **characterized in that** the reactants are present on commencement of the reaction in a molar ratio of first component to second component of 1:0.11 to 1:2000.

7. Process according to at least one of the preceding claims, **characterized in that**, based on the overall reaction mixture, the maximum total amount of any solvent present is less than 20% by weight, preferably less than 10% by weight, especially less than 5% by weight.

8. Process according to at least one of the preceding claims, **characterized in that** it is conducted under anhydrous conditions.

## Revendications

1. Procédé pour la préparation de sphingolipides de formule générale I 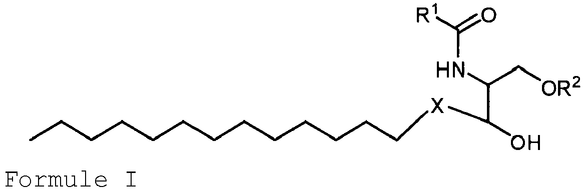 par transformation d'un premier composant, un lysosphingolipide de formule générale II, 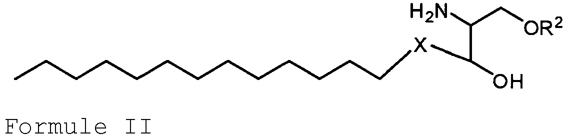 avec un deuxième composant, un donneur de groupements acyle du groupement acyle R¹CO, où
R¹ représente une chaîne alkyle comprenant 2 à 55 atomes de carbone, linéaire ou ramifiée, contenant le cas échéant un(e) ou plusieurs liaisons multiples et/ou cycles aromatiques ou hétéroaromatiques, le cas échéant interrompue par des atomes d'oxygène ou des fonctionnalités ester ou amide, le cas échéant substituée par au moins un autre groupement choisi parmi les groupements alkylhydroxy, céto ou amine,
R² représente H, phosphocholine, sérine, éthanolamine ou un sucre, de préférence un sucre ou H, de manière particulièrement préférée H et
X représente CH=CH, CH₂-CH₂ ou CH₂-HCOH, de préférence CH₂-CH₂,
**caractérisé en ce que**, par rapport à la charge réactionnelle totale, le premier et le deuxième composant représentent, au total, au moins 70% en poids, de préférence 90% en poids, en particulier 95% en poids et on n'utilise, dans la charge réactionnelle totale, pas plus de 600 unités de laurate de propyle d'au moins une hydrolase d'ester carboxylique de la classe d'enzymes 3.1.1 par gramme de premier composant, une unité de laurate de propyle étant définie comme la quantité d'enzyme qui synthétise une µmole de laurate de propyle par minute à partir de 1-propanol et d'acide laurique et **caractérisé en ce que** l'hydrolase d'ester carboxylique est choisie dans le groupe comprenant la lipase de Thermomyces lanuginosus présentant le numéro d'enregistrement O59952, les lipases A et B présentant le numéro d'enregistrement P41365 de Candida antarctica et la lipase de Mucor miehei présentant le numéro d'enregistrement P19515, la lipase d'Humicola sp. présentant le numéro d'enregistrement O59952, la lipase de Rhizomucor javanicus présentant le numéro d'enregistrement S32492, la lipase de Rhizopus oryzae présentant le numéro d'enregistrement P61872, les lipases de Candida rugosa présentant les numéros d'enregistrement P20261, P32946, P32947, P3294 et P32949, la lipase de Rhizopus niveus présentant le numéro d'enregistrement P61871, la lipase de Penicillium camemberti présentant le numéro d'enregistrement P25234, les lipases d'Aspergillus niger présentant les numéros d'enregistrement ABG73613, ABG73614 et ABG37906 et la lipase de Penicillium cyclopium présentant le numéro d'enregistrement P61869, ainsi que leurs homologues, sur le plan des acides aminés, à raison d'au moins 60%, de préférence d'au moins 80%, préférablement d'au moins 90%, de manière particulièrement préférée d'au moins 95%, 98% ou 99%, les numéros d'enregistrement correspondant à ceux des entrées dans la base de données ProteinBank du NCBI en date du 01.01.2017.

2. Procédé selon la revendication 1, **caractérisé en ce qu'**on utilise, comme premier composant, de la sphinganine présentant R² = H et X = CH₂-CH₂.

3. Procédé selon la revendication 1 ou 2, **caractérisé en ce que** le deuxième composant est choisi parmi les donneurs de groupements acyle, qui mettent à disposition un groupement acyle du groupe des groupements acyle d'acides gras naturels.

4. Procédé selon au moins l'une quelconque des revendications précédentes, **caractérisé en ce que** le deuxième composant est choisi parmi les esters à base d'alcanols et de polyols comprenant jusqu'à 6 atomes de carbone, de manière particulièrement préférée jusqu'à 3 atomes de carbone, de manière tout particulièrement préférée parmi les esters de glycérol.

5. Procédé selon au moins l'une quelconque des revendications précédentes, **caractérisé en ce qu'**on utilise, comme premier composant, de la sphinganine présentant R² = H et X = CH₂-CH₂ et, comme deuxième composant, un donneur de groupements acyle choisis dans le groupe comprenant la graisse de coco, l'huile de palmiste, l'huile d'olive, l'huile de palme, l'huile d'argan, l'huile de ricin, l'huile de lin et l'huile de babassu.

6. Procédé selon au moins l'une quelconque des revendications précédentes, **caractérisé en ce que** les réactifs, au début de la transformation, se trouvent dans un rapport molaire du premier au deuxième composant de 1:0,11 à 1:2000.

7. Procédé selon au moins l'une quelconque des revendications précédentes, **caractérisé en ce que**, par rapport à la totalité de la charge réactionnelle, au total, un solvant est contenu au maximum à raison de moins de 20% en poids, de préférence de moins de 10% en poids, en particulier de moins de 5% en poids.

8. Procédé selon au moins l'une quelconque des revendications précédentes, **caractérisé en ce qu'**il est réalisé dans des conditions anhydres.
